# EUROPEAN PATENT SPECIFICATION

(11) **EP 2 736 505 B1**
(45) Date of publication and mention of the grant of the patent: **27.09.2017**
(21) Application number: 12820828.7
(22) Date of filing: 13.07.2012
(51) Int. Cl.: A61K 31/196, A61P 3/06

(54) **3-HYDROXYANTHRANILIC ACID (3-HAA) THERAPY FOR PREVENTION AND TREATMENT OF HYPERLIPIDEMIA AND ITS CARDIOVASCULAR COMPLICATIONS**
THERAPIE MIT 3-HYDROXYANTHRANILSÄURE (3-HAA) ZUR VORBEUGUNG UND BEHANDLUNG VON HYPERLIPIDÄMIE UND IHREN KARDIOVASKULÄREN KOMPLIKATIONEN
THÉRAPIE AVEC L'ACIDE 3-HYDROXYANTHRANILIQUE (3-HAA) POUR LA PRÉVENTION ET LE TRAITEMENT DE L'HYPERLIPIDÉMIE ET SES COMPLICATIONS CARDIOVASCULAIRES

(30) Priority: 29.07.2011 SE 1150728
(43) Date of publication of application: 04.06.2014
(73) Proprietor: Hansson, Göran K., 112 39 Stockholm (SE)
(72) Inventor: KETELHUTH, Daniel, 126 28 Stockholm (SE)
(74) Representative: Awapatent AB
(86) International application number: PCT/SE2012/050835
(87) International publication number: WO 2013/019156

(56) References cited:
- WO-A1-2006/122353
- US-A- 2 652 332
- Lei Zhang ET AL: "The tryptophan metabolite 3-hydroxyanthranilic acid lowers plasma lipids and decreases atherosclerosis in hypercholesterolaemic mice", European heart journal, 1 August 2012 (2012-08-01), page 2025, XP055141918, England DOI: 10.1093/eurheartj/ehs175 Retrieved from the Internet: URL:http://www.ncbi.nlm.nih.gov/pubmed/227 11758
- CHRISTEN, S. ET AL.: "Inhibition by interferon-gamma of human mononuclear cell-mediated low density lipoprotein oxidation", J.CLIN.INVEST., vol. 93, May 1994 (1994-05), pages 2149-2158, XP002732100,
- L GAIL DARLINGTON ET AL: "On the Biological Importance of the 3-hydroxyanthranilic Acid: Anthranilic Acid Ratio Introduction", INTERNATIONAL JOURNAL OF TRYPTOPHAN RESEARCH, vol. 3, no. 2010, 1 January 2010 (2010-01-01), pages 51-593, XP055141921,
- ZHANG, LEI ET AL.: 'The tryptophan metabolite 3- hydroxyanthranlic acid lowers plasma lipids and decreases atherosclerosis in hypercholesterolaemic mice' EUROPEAN HEART JOURNAL vol. 33, 18 June 2012, pages 2025 - 2034, XP055141918
- PAWLAK, KRYSTYNA ET AL.: '3-hydroxyanthranilic acid is independently associated with monocyte chemoattractant protein-1 (CCL2) and macrophage inflammatory protein 1-beta (CCL4) in patients with chronic kidney disease' CLINICAL BIOCHEMISTRY vol. 43, 2010, pages 1101 - 1106, XP027248979
- ALLEGRI GRAZIELLA ET AL.: 'Tryptophan metabolism along the kynurenine pathway in diet-induced and genetic hypercholesterolemic rabbits' CLINICA CHIMICA ACTA vol. 350, no. 1-2, 2004, pages 41 - 49, XP004629808
- RAGAZZI EUGENIO ET AL.: 'Enzyme activities along the tryptophan-nicotinic acid pathway in alloxan diabetic rabbits' BIOCHIMICA ET BIOPHYSICA ACTA vol. 1571, 2002, pages 9 - 17, XP002727213
- RUDZITE, V. ET AL.: 'Changes in membrane fluidity induced by thryptophan and its metabolites' ADVANCES IN EXPERIMENTAL MEDICINE AND BIOLOGY vol. 467, 1999, pages 353 - 367, XP008172314
- THOMAS, SHANE R. ET AL.: 'Antioxidant activities and redox regulation of interferon-gamma-induced tryptophan metabolism in human monocytes and macrophages' ADVANCES IN EXPERIMENTAL MEDICINE AND BIOLOGY vol. 467, 1999, pages 541 - 552, XP008172313
- DARLINGTON, L. GAIL ET AL.: 'On the biological importance of the 3- hydroxyanthranilic acid:anthranilic acid ratio' INTERNATIONAL JOURNAL OF TRYPTOPHAN RESEARCH vol. 3, 2010, pages 51 - 59, XP055141921
- BROUNS, RAF. ET AL.: 'The role of tryptophan catabolism along the kynurenine pathway in acute ischemic stroke' NEUROCHEMICAL RESEARCH vol. 35, no. 9, 2010, pages 1315 - 1322, XP019827379
- GREGORY GILES I. ET AL.: 'Electrochemical and in vitro evaluation of the redox-properties of kynurenine species' BIOCHEMICAL AND BIOPHYSICAL RESEARCH COMMUNICATIONS vol. 300, no. 3, 2003, pages 719 - 724, XP055141923
- DARLINGTON, L.G. ET AL.: 'Altered kynurenine metabolism correlates with infarct volume in stroke' EUROPEAN JOURNAL OF NEUROSCIENCE vol. 26, no. 8, October 2007, pages 2211 - 2221, XP055141928

## Description

### FIELD OF THE INVENTION

The present invention relates to the use of the tryptophan metabolite 3-hydroxyanthranilic acid (3-HAA) for treatment of hyperlipidemia. In particular the invention includes: the use of 3-HAA, as such or together with a suitable vehicle, as a lipid-lowering therapy for prevention and/or treatment of hyperlipidemia and the cardiovascular complications associated with hyperlipidemia, specifically atheroma formation, myocardial infarction, ischemic stroke and transitory ischemic attacks, renal impairment, aortic aneurysms and critical limb ischemia caused by atherosclerosis.

### BACKGROUND OF THE INVENTION

Cardiovascular diseases (CVD), largely caused by formation of atherosclerotic atheroma in the arteries, are the main cause of death in the Western world and also increasingly in developing countries ¹. However, progress in prevention of hyperlipidemia using lipid-lowering drugs, e.g. statins, has a proven survival benefit for both primary prevention (i.e. in individuals without clinical evidence of CVD) and secondary prevention (i.e. in patients with established CVD) ²⁻⁴ Additionally, other classes of drugs, e.g. fibrates, which raise HDL levels, have been shown to reduce the progression of coronary disease in clinical trials ^{5,6} and to reduce the incidence of cardiovascular events in outcome studies ^{7,8}.

Atherosclerosis is a chronic inflammatory condition initiated by retention and accumulation of apolipoprotein B100 (ApoB100)-containing lipoproteins, in particular Low density lipoprotein (LDL), in the artery wall leading to a maladaptive set of responses of macrophages and T cells and the formation of atheroma in the arteries ^{9,10}. Therefore, lowering circulating ApoB100 lipoproteins may have other beneficial effects in addition to decreasing the probability of lipids to accumulate in the arterial wall.

High levels of LDL and its precursor particle, very-low-density lipoprotein (VLDL), are associated with an increased risk for myocardial infarction, stroke, and other complications of atheroma. Elevated LDL and VLDL levels are reflected in high cholesterol and triglyceride levels in samples of blood serum or plasma. Such elevated blood lipid levels constitute the condition of hyperlipidemia which, as mentioned, is associated with increased risk for myocardial infarction, stroke and other atherosclerotic complications.

Hyperlipidemic levels are defined in the SCORE criteria of the European Society of Cardiology, which also identify target levels that should be reached by lipid-lowering therapy ¹¹. The cardiovascular risk impacted by high LDL cholesterol or total cholesterol is lowered when HDL cholesterol levels are high, as outlined in the same publication. Vice versa, low HDL levels increase the cardiovascular risk of elevated total cholesterol or LDL cholesterol. Current levels for hyperlipidemia as defined by the Swedish Medical Products Agency (Läkemedelsverket) are: Total cholesterol > 5 mmol/L, LDL-cholesterol >3 mmol/L, and HDL-cholesterol <1 mmol/L¹².

HDL particles exert their protective effect on arteries by mediating the removal of cholesterol from cells. After incorporation into HDL, cholesterol molecules are transported to the liver, converted into bile acids, and eliminated through the intestines.

Current therapy for hyperlipidemia is dominated by the group of drugs called statins. These compounds have good effects on LDL cholesterol in most individuals but only minor effects on HDL¹³. Furthermore, some individuals do not tolerate statins or other lipid-lowering drugs. For these reasons, there is a need for development of new lipid-lowering agents.

Modified phospholipids of LDL, such as lysophosphatidylcholine and 1-palmitoyl-2-(5'-oxovaleroyl)-sn-glycero-3-phosphocholine (POVPC), can stimulate endothelial cells, smooth muscle cells, and macrophages^{14,15}. It is now known that such atherogenic lipids initiate innate immune response through activation of Toll-like receptors ¹⁶⁻¹⁸. Furthermore, cholesterol accumulating in macrophages that have internalized LDL particles can form microcrystals that directly activate the inflammasome, leading to production of the proinflammatory cytokine, interleukin-1beta ¹⁸. Collectively, these data imply that modification of LDL generates endogenous ligands that trigger activation of innate immune responses that can promote atheroma formation.

Adaptive immunity also plays a key role in the development of atherosclerotic atheroma. Antigen-presenting cells encounter and internalize antigens in the intima, including LDL particles that are internalized through scavenger receptors ¹⁹. After proteolysis, fragments of the LDL protein ApoB100 associate intracellularly with MHC class II proteins, traffic to the cell surface and are presented to T cells. The latter event leads to T cell activation and the production of cytokines which will trigger and maintain local inflammation ²⁰.

In addition to their effects on the local pathological process, immune cells and mediators modulate lipid metabolism on the systemic level. Thus, the proinflammatory cytokine tumor necrosis factor (TNF) inhibits lipoprotein lipase, a key enzyme in triglyceride metabolism, leading to hypertriglyceridemia ²¹. Another TNF-like protein, LIGHT, inhibits another lipase, hepatic lipase, by acting in the liver ^{22, 23}. This leads to reduced triglyceride catabolism and accumulation in the blood of large lipoproteins that contain triglycerides and cholesterol. As a third example, the cholesterol-lowering drugs of the statin class exert significant immunomodulatory effects by reducing T cell activation and inhibiting several autoimmune diseases^{24, 25}.

All these data demonstrate an intricate crosstalk between the immune and metabolic systems and suggest that it might be possible to treat hyperlipidemia and prevent atheroma formation by using immune cell-dependent pathways.

Recent data shows that IDO (Indoleamine 2,3-Dioxygenase) and IDO-catalyzed tryptophan metabolism play critical roles in the induction of immune suppression and tolerance (reviewed in ²⁶). IDO is coded by the IDO1 gene (encoded by 10 exons in the chromosome 8, 8p12-p11 in humans). The enzyme metabolizes L-Tryptophan (L-Trp) into N-formylkynurenine, a product that is rapidly converted by formamidase to L-kynurenine (KYN), which in turn can either enter the bloodstream or be further metabolized to downstream Kynurenines (Kyns). These Kyns include 3-hydroxykynurenine (3-HK), 3-hydroxyanthranilic acid (3-HAA), and quinolinic acid (QUIN) ²⁷.

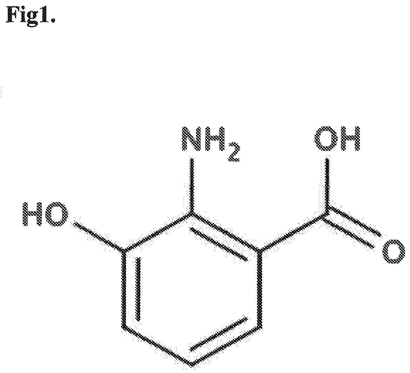

It has been shown that downstream tryptophan metabolites such as 3-hydroxyanthranillic acid (3-HAA, Fig1) can inhibit the function of Th1 and Th2 cells and increase the percentage of regulatory T cells (Tregs). Further, administration of 3-HAA has been shown to decrease inflammation induced by Th17 cells and protect mice against autoimmune encephalitis ^{28, 29}. In fact, data suggests that 3-HAA could control autoimmunity by direct effects on T cells or by indirect effects through altered antigen presentation on dendritic cells and macrophages ³⁰.

### SUMMARY OF THE INVENTION

Hyperlipidemia is the condition of abnormally elevated levels of blood lipids, in particular cholesterol and triglycerides transported in the plasma lipoproteins LDL and VLDL. High lipid levels are followed by sub-endothelial retention and accumulation of LDL in the artery wall; this leads to a chronic maladaptive inflammatory response of macrophages and T cells and to atheroma formation. In this context, prevention of hyperlipidemia using lipid-lowering drugs, e.g. statins and fibrates has proven to decrease the number of cardiovascular events and increase the survival of patients at risk or with established CVD^{2-4, 7, 8}

The present inventors found that the immunomodulatory compound 3-HAA demonstrated an unexpected potent effect against hyperlipidemia and significantly reduced total plasma cholesterol and triglyceride levels in a series of experiments. Besides, 3-HAA significantly raised HDL levels and decreased VLDL/HDL and LDL/HDL ratios. Hence, the beneficial changes in plasma lipids were accompanied by a significant reduction in atherosclerotic plaque development. Therefore, 3-HAA can be recognized as a new lipid-lowering agent for the prevention and treatment of hyperlipidemia and its cardiovascular complications, i.e. atheroma formation, myocardial infarction, ischemic stroke and transient ischemic attacks, renal impairment, aortic aneurysms and critical limb ischemia caused by atherosclerosis.

Thus, the present invention relates to use of 3-HAA in the treatment of hyperlipidemia or in the prevention of a cardiovascular complication of hyperlipidemia.

According to one embodiment, the hyperlipidemia is selected from the group consisting of hypercholesterolemia, hypertriglyceridemia and combined (forms of) hyperlipidemia. In a preferred embodiment the hyperlipidemia is associated with low levels of high-density lipoprotein (HDL) in plasma.

In a preferred embodiment the cardiovascular complication of hyperlipidemia is atheroma formation.

In a further embodiment of the present invention, the cardiovascular complication of hyperlipidemia is a clinical manifestation of atheroma formation.

According to one embodiment the 3-HAA is used for prevention of myocardial infarction and/or heart failure.

In one embodiment the 3-HAA is for use for prevention of angina pectoris.

In a preferred embodiment the 3-HAA is for use for prevention of ischemic stroke and/or transient ischemic attacks.

In another embodiment the 3-HAA is for use for prevention of peripheral ischemia, gangrene, renal impairment, aortic aneurysms, and/or critical limb ischemia.

According to one embodiment of the present invention, the complication of hyperlipidemia is a dermatological complication of hyperlipidemia.

According to another embodiment, the dermatological complication of hyperlipidemia is xanthomas.

### Brief description of the drawings

**Fig1****. Molecular representation of 3-Hydroxy anthranilic acid (3-HAA)**
   Molecular formula: C₇H₇NO₃. Molar mass 153.14 g/mol. IIUPAC name: 2-Amino-3-hydroxybenzoic acid.
**Fig2****. Uptake of FITC-oxLDL by peritoneal macrophages**
   Peritoneal macrophages from *Ldlr*-/- mice injected with 3-HAA (200 mg/Kg) or PBS (8 weeks treatment) were incubated with FITC-oxLDL at a concentration of 20 µg protein/ml for 2h at 37°C. The uptake was quantified by flow cytometry. (A) Values given are MFI of FITC-oxLDL, means ± SE of triplicate wells for each mouse (n= 5 and 6, for 3-HAA or PBS respectively). (B) The graph shows representative histograms from peritoneal macrophages from 3-HAA or PBS treated mice. **) P<0.01.

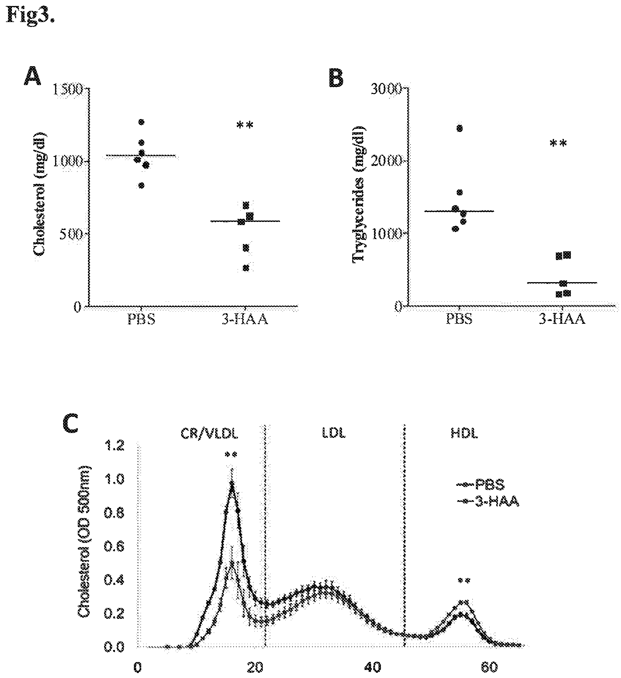

**Fig3****. Plasma lipid analysis**
   Levels of (A) total cholesterol and (B) triglycerides were evaluated in plasma from 3-HAA or PBS treated mice (8 weeks treatment). (C) Size analysis of lipoprotein profiles from plasma of 3-HAA or PBS treated mice. The cholesterol concentration in each fraction (y axis) is plotted against retention fraction number (x axis); curves show mean ± SEM for 3-HAA and PBS treated mice. Values are expressed as mean ± SEM (n= 5 and 6, for 3-HAA or PBS respectively). **) P<0.01.

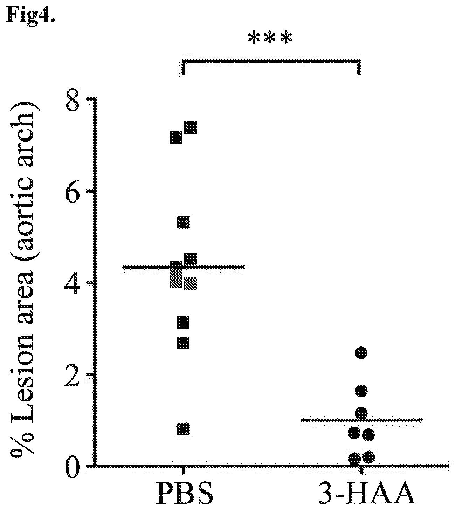

**Fig4****. 3-HAA reduces the development of atheroma**
   Twelve weeks old *Ldlr*-/- mice were treated with 3-HAA or PBS as control. The mice were sacrificed after 8 weeks on a Western diet. Dissected arches were stained with Sudan IV *en* face and % lesion area of total vessel area was calculated using Image J image analysis software (NIH, Bethesda, MD). The additive area of all the plaques in a given aortic arch was calculated as a percent of the total surface area of the arch. (n= 7 and 10, for 3-HAA or PBS respectively). ***) P<0.001.

### EXPERIMENTAL

### Procedure

It was hypothesized that induction of protective immunity, with effects on metabolism and inflammation, could be achieved by injecting 3-HAA (200 mg/kg) intraperitoneally, 3 times per week, for 8 weeks. Twelve weeks old *Ldlr*-/- mice were treated with 3-HAA, or PBS as control. The mice were fed a high-fat diet starting 2 days after the first injection until sacrifice, 8 weeks later with CO₂.

Plasma cholesterol and triglycerides were measured using enzymatic colorimetric kits according to the manufacturer's protocol. Dissected aortic arches were stained with Sudan IV *en face* and % lesion area of total vessel area was calculated. Additionally, the effect of 3-HAA on the uptake oxLDL was evaluated in cultures of peritoneal macrophages from 3-HAA or PBS treated mice.

### MATERIAL AND METHODS

### Animals and animal treatments

*Ldlr*-/- mice were bred and housed in the Animal Experimentation Unit at the Center for Molecular Medicine, Karolinska University Hospital. The mice (n= 7-10 per group) received 3 intra-peritoneal injections of PBS (200 µl) or 3-HAA (200 mg/Kg in PBS) per week for 6 weeks and 1 injection per week in the following two weeks (total 24 injections) and were sacrificed with CO₂. The mice were fed a high-fat diet (corn starch, cocoa butter, casein, glucose, sucrose, cellulose flour, minerals and vitamins; 17.2% protein, 21% fat (62.9% saturated, 33.9 unsaturated and 3.4% polyunsaturated), 0.15% cholesterol, 43% carbohydrates, 10% H₂O and 3.9% cellulose fibers; R638 Lantmännen, Sweden) starting 2 days after the start of treatment.

### Low-density lipoprotein (LDL) isolation

LDL (d = 1.019 - 1.063 g/mL) was isolated from pooled plasma of healthy donors by ultracentrifugation, as described ³¹. 2mM Benzamidine, 0.5mM PMSF and 0.1U/ml Aprotinin were added immediately after the plasma was prepared and LDL was dialyzed extensively against PBS after isolation. One millimolar EDTA was added to an aliquot of LDL to prevent modification of LDL particles.

### Preparation of FITC (fluorescein isothiocyanate) labeled oxidized LDL (FITC-oxLDL)

LDL was labeled using a modification of a previously described method ³². Briefly, LDL (1.5 - 2 mg/ml) was dialyzed overnight against 500 mM NaHC0₃ pH 9.5. Next, 50 µg of FITC (Sigma-Aldrich, St.Louis, MO, USA), dissolved in DMSO (1 mg/ml), was added for each mg of protein in LDL, and incubated at room temperature for 2 hours. After incubation conjugates were separated from the free fluorochrome by gel filtration using a PD10 column (GE Healthcare, Uppsala, Sweden) and PBS for elution. FITC conjugation was evaluated by absorption spectroscopy against a FITC standard curve at 495 nm. Protein concentration was determined by Bradford assay (Biorad, CA, USA). Oxidized FITC-LDL (FITC-oxLDL) was obtained by incubating 1 ml of FITC-LDL (1 mg/ml ) in the presence of 20 µM CuSO₄ for 18 h at 37°C. The extent of oxidation was evaluated by the TBARS assay, as described ³³.

### OxLDL uptake by peritoneal macrophages

Twenty four hours after the last injection of 3-HAA or PBS, peritoneal macrophages were isolated from *Ldlr*-/- mice by peritoneal lavage with PBS. After 2 to 3 hours in PBS, the macrophages were plated in 96-well plates at a density of 1x10⁵ cells per well with 20 µg/ml FITC-oxLDL in RPMI 1640 medium containing 1% FCS. After 2 h incubation at 37°C cells were washed twice with PBS and fixed with 4% paraformaldehyde in PBS. The cells were analyzed on a CyAn^{™} ADP flow cytometer (Dako, Glostrup, Denmark).

### Plasma lipid analysis

Plasma cholesterol and triglycerides were measured using enzymatic colorimetric kits (Randox Lab. Ltd. Crumin, UK) according to the manufacturer's protocol.

### Lipoprotein profiles

Plasma cholesterol lipoprotein profiles were determined using a modification of the method of Okazaki et al ³⁴. Briefly, plasma samples (50 µl) from mice treated with 3-HAA or PBS were fractionated using an HR10/30 Superose 6 column (GE Healthcare, Uppsala, Sweden) and a Discovery BIO GFC-500 as pre-column (5 cm x 7.8 i.d.; Supelco^{®},Sigma-Aldrich, PA, USA) coupled to Prominence UFLC system (Shimadzu, Kyoto, Japan) and equilibrated with Tris-buffered saline, pH 7.4. Fractions of 200 µl were collected using Foxy Jr^{®} fraction collector (Teledyne Isco Inc, NE, USA) and total cholesterol was determined in each fraction using enzymatic colorimetric kit (Randox Lab. Ltd. Crumin, UK).

### Lesion analysis

*En face* lipid accumulation was determined in the aortic arch from 3-HAA and PBS treated mice using Sudan IV staining. Briefly, dissected arches were fixed in 4% neutral buffered formalin. Samples were then cut longitudinally, splayed, pinned and subjected to Sudan IV staining (red color). Images were captured using a Leica DC480 camera connected to a Leica MZ6 stereo microscope (Leica, Wetzlar, Germany). The additive area of all the plaques in a given aortic arch was calculated as a percent of the total surface area of the arch (not including branching vessels). Quantitation of plaques was performed using Image J software (NIH, Bethesda, USA).

### Statistical analysis

Values are expressed as mean ± standard error of the mean (SEM) unless otherwise indicated. The nonparametric Mann-Whitney U test was used for comparisons between groups. Differences were considered significant at P values below 0.05.

### RESULTS

### 3-hydroxyanthranilic acid inhibits oxLDL uptake

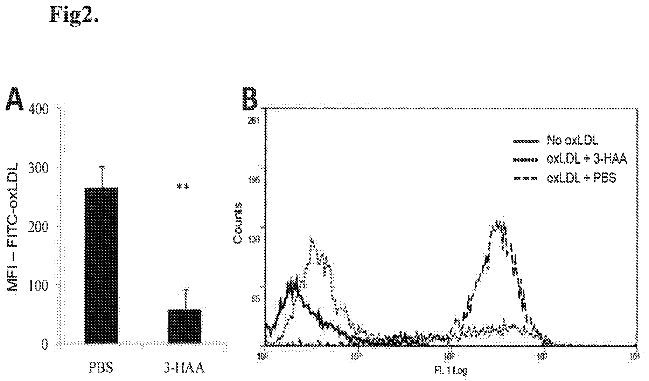

The uptake of fluorescent FITC-oxLDL was quantified by flow cytometry as described in the Methods section. As shown in Fig.2, peritoneal macrophages from mice treated with 3-HAA displayed approx 79% reduced uptake of FITC-oxLDL. Therefore, 3-HAA inhibits uptake of modified LDL particles, the key event in foam cell formation.

### 3-hydroxyanthranilic acid has a potent lipid-lowering effect

Unexpected for an immunomodulatory compound, administration of 3-HAA to *Ldlr*-/- mice was associated with a marked, statistically significant reduction in plasma total cholesterol (approx 50%) and triglycerides (approx 72%) (Fig.3). Additionally, 3-HAA effectively raised HDL levels and reduced VLDL/HDL (4.0 ± 0.55 and 1.4 ± 0.31, mean ± SEM of PBS and 3-HAA treated mice respectively; P< 0.01) and LDL/HDL ratios (2.3 ± 0.24 and 1.4 ± 0.16, mean ± SEM of PBS and 3-HAA treated mice respectively; P<0.05). These data identify 3-HAA as a new type of lipid-lowering agent. 3-HAA administration had no effect on body weight (data not shown).

### Reduction of plasma lipids induced by 3-hydroxyanthranilic acid leads to reduced atheroma formation

Finally, we examined whether 3-HAA could affect atheroma formation. *Ldlr*-/- mice, which develop atheromatous lesions when treated with high fat diet, were treated with 3-HAA for 8 weeks. The dramatic reduction in total plasma cholesterol and triglyceride levels led to an approximately 90 % reduction in atherosclerotic lesion area in *en face* preparations of the aortic arch (Fig.4).

### REFERENCES

1. Rosamond W, Flegal K, Friday G, Furie K, Go A, Greenlund K, Haase N, Ho M, Howard V, Kissela B, Kittner S, Lloyd-Jones D, McDermott M, Meigs J, Moy C, Nichol G, O'Donnell CJ, Roger V, Rumsfeld J, Sorlie P, Steinberger J, Thom T, Wasserthiel-Smoller S, Hong Y. Heart disease and stroke statistics--2007 update: A report from the american heart association statistics committee and stroke statistics subcommittee. Circulation. 2007;115:e69-171
2. Baigent C, Keech A, Kearney PM, Blackwell L, Buck G, Pollicino C, Kirby A, Sourjina T, Peto R, Collins R, Simes R. Efficacy and safety of cholesterol-lowering treatment: Prospective meta-analysis of data from 90,056 participants in 14 randomised trials of statins. Lancet. 2005; 366:1267-1278
3. Cannon CP, Steinberg BA, Murphy SA, Mega JL, Braunwald E. Meta-analysis of cardiovascular outcomes trials comparing intensive versus moderate statin therapy. J Am Coll Cardiol. 2006;48:438-445
4. Leeper NJ, Ardehali R, deGoma EM, Heidenreich PA. Statin use in patients with extremely low low-density lipoprotein levels is associated with improved survival. Circulation. 2007;116:613-618
5. Ruotolo G, Ericsson CG, Tettamanti C, Karpe F, Grip L, Svane B, Nilsson J, de Faire U, Hamsten A. Treatment effects on serum lipoprotein lipids, apolipoproteins and low density lipoprotein particle size and relationships of lipoprotein variables to progression of coronary artery disease in the bezafibrate coronary atherosclerosis intervention trial (becait). J Am Coll Cardiol. 1998;32:1648-1656
6. Ericsson CG, Nilsson J, Grip L, Svane B, Hamsten A. Effect of bezafibrate treatment over five years on coronary plaques causing 20% to 50% diameter narrowing (the bezafibrate coronary atherosclerosis intervention trial [becait]). Am J Cardiol. 1997;80:1125-1129
7. Boden WE. High-density lipoprotein cholesterol as an independent risk factor in cardiovascular disease: Assessing the data from framingham to the veterans affairs high--density lipoprotein intervention trial. Am J Cardiol. 2000;86:19L-22L
8. Rubins HB, Robins SJ, Collins D, Fye CL, Anderson JW, Elam MB, Faas FH, Linares E, Schaefer EJ, Schectman G, Wilt TJ, Wittes J. Gemfibrozil for the secondary prevention of coronary heart disease in men with low levels of high-density lipoprotein cholesterol. Veterans affairs high-density lipoprotein cholesterol intervention trial study group. N Engl J Med. 1999;341:410-418
9. Tabas I, Williams KJ, Boren J. Subendothelial lipoprotein retention as the initiating process in atherosclerosis: Update and therapeutic implications. Circulation. 2007;116:1832-1844
10. Hansson GK. Inflammation, atherosclerosis, and coronary artery disease. The New England journal of medicine. 2005;352:1685-1695
11. Reiner Z, Catapano AL, De Backer G, Graham I, Taskinen MR, Wiklund O, Agewall S, Alegria E, Chapman MJ, Durrington P, Erdine S, Halcox J, Hobbs R, Kjekshus J, Filardi PP, Riccardi G, Storey RF, Wood D, Bax J, Vahanian A, Auricchio A, Baumgartner H, Ceconi C, Dean V, Deaton C, Fagard R, Filippatos G, Funck-Brentano C, Hasdai D, Hoes A, Kearney P, Knuuti J, Kolh P, McDonagh T, Moulin C, Poldermans D, Popescu BA, Sechtem U, Sirnes PA, Tendera M, Torbicki A, Vardas P, Widimsky P, Windecker S, Reviewers D, Berkenboom G, De Graaf J, Descamps O, Gotcheva N, Griffith K, Guida GF, Gulec S, Henkin Y, Huber K, Kesaniemi YA, Lekakis J, Manolis AJ, Marques-Vidal P, Masana L, McMurray J, Mendes M, Pagava Z, Pedersen T, Prescott E, Rato Q, Rosano G, Sans S, Stalenhoef A, Tokgozoglu L, Viigimaa M, Wittekoek ME, Zamorano JL. Esc/eas guidelines for the management of dyslipidaemias: The task force for the management of dyslipidaemias of the european society of cardiology (esc) and the european atherosclerosis society (eas). Eur Heart J. 2011; 32:1769-1818
12. Läkemedelsverket. Förebyggande av aterosklerotisk hjärtkärlsjukdom. Information från läkemedelsverket. 2006;3
13. Barter P, Gotto AM, LaRosa JC, Maroni J, Szarek M, Grundy SM, Kastelein JJ, Bittner V, Fruchart JC. Hdl cholesterol, very low levels of Idl cholesterol, and cardiovascular events. N Engl J Med. 2007; 357:1301-1310
14. Watson AD, Leitinger N, Navab M, Faull KF, Horkko S, Witztum JL, Palinski W, Schwenke D, Salomon RG, Sha W, Subbanagounder G, Fogelman AM, Berliner JA. Structural identification by mass spectrometry of oxidized phospholipids in minimally oxidized low density lipoprotein that induce monocyte/endothelial interactions and evidence for their presence in vivo. J Biol Chem. 1997;272:13597-13607
15. Gharavi NM, Alva JA, Mouillesseaux KP, Lai C, Yeh M, Yeung W, Johnson J, Szeto WL, Hong L, Fishbein M, Wei L, Pfeffer LM, Berliner JA. Role of the jak/stat pathway in the regulation of interleukin-8 transcription by oxidized phospholipids in vitro and in atherosclerosis in vivo. The Journal of biological chemistry. 2007;282:31460-31468
16. Seimon TA, Nadolski MJ, Liao X, Magallon J, Nguyen M, Feric NT, Koschinsky ML, Harkewicz R, Witztum JL, Tsimikas S, Golenbock D, Moore KJ, Tabas I. Atherogenic lipids and lipoproteins trigger cd36-tlr2-dependent apoptosis in macrophages undergoing endoplasmic reticulum stress. Cell metabolism. 2010;12:467-482
17. Stewart CR, Stuart LM, Wilkinson K, van Gils JM, Deng J, Halle A, Rayner KJ, Boyer L, Zhong R, Frazier WA, Lacy-Hulbert A, El Khoury J, Golenbock DT, Moore KJ. Cd36 ligands promote sterile inflammation through assembly of a toll-like receptor 4 and 6 heterodimer. Nat Immunol. 2010;11:155-161
18. Duewell P, Kono H, Rayner KJ, Sirois CM, Vladimer G, Bauernfeind FG, Abela GS, Franchi L, Nunez G, Schnurr M, Espevik T, Lien E, Fitzgerald KA, Rock KL, Moore KJ, Wright SD, Hornung V, Latz E. Nlrp3 inflammasomes are required for atherogenesis and activated by cholesterol crystals. Nature. 2010;464:1357-1361
19. Andersson J, Libby P, Hansson GK. Adaptive immunity and atherosclerosis. Clin Immunol. 2010;134:33-46
20. Libby P, Ridker PM, Hansson GK. Progress and challenges in translating the biology of atherosclerosis. Nature. 2011;473:317-325
21. Beutler B, Greenwald D, Hulmes JD, Chang M, Pan YC, Mathison J, Ulevitch R, Cerami A. Identity of tumour necrosis factor and the macrophage-secreted factor cachectin. Nature. 1985;316:552-554
22. Lo JC, Wang Y, Tumanov AV, Bamji M, Yao Z, Reardon CA, Getz GS, Fu YX. Lymphotoxin beta receptor-dependent control of lipid homeostasis. Science. 2007;316:285-288
23. Hansson GK. Medicine. Light hits the liver. Science. 2007;316:206-207
24. Youssef S, Stuve O, Patarroyo JC, Ruiz PJ, Radosevich JL, Hur EM, Bravo M, Mitchell DJ, Sobel RA, Steinman L, Zamvil SS. The hmg-coa reductase inhibitor, atorvastatin, promotes a th2 bias and reverses paralysis in central nervous system autoimmune disease. Nature. 2002;420:78-84
25. McCarey DW, McInnes IB, Madhok R, Hampson R, Scherbakov O, Ford I, Capell HA, Sattar N. Trial of atorvastatin in rheumatoid arthritis (tara): Double-blind, randomised placebo-controlled trial. Lancet. 2004;363:2015-2021
26. Grohmann U, Bronte V. Control of immune response by amino acid metabolism. Immunological reviews. 2010;236:243-264
27. Stone TW, Darlington LG. Endogenous kynurenines as targets for drug discovery and development. Nat Rev Drug Discov. 2002;1:609-620
28. Platten M, Ho PP, Youssef S, Fontoura P, Garren H, Hur EM, Gupta R, Lee LY, Kidd BA, Robinson WH, Sobel RA, Selley ML, Steinman L. Treatment of autoimmune neuroinflammation with a synthetic tryptophan metabolite. Science. 2005;310:850-855
29. Yan YP, Zhang GX, Gran B, Fallarino F, Yu S, Li HM, Cullimore ML, Rostami A, Xu H. Ido upregulates regulatory t cells via tryptophan catabolite and suppresses encephalitogenic t cell responses in experimental autoimmune encephalomyelitis. Journal of Immunology. 2010;185:5953-5961
30. Munn DH, Mellor AL. Indoleamine 2,3-dioxygenase and tumor-induced tolerance. Journal of Clinical Investigation. 2007; 117:1147-1154
31. Havel RJ, Eder HA, Bragdon JH. The distribution and chemical composition of ultracentrifugally separated lipoproteins in human serum. J Clin Invest. 1955;34:1345-1353
32. Schmitz G, Wulf G, Bruning T, Assmann G. Flow-cytometric determination of high-density-lipoprotein binding sites on human leukocytes. Clin Chem. 1987;33:2195-2203
33. Puhl H, Waeg G, Esterbauer H. Methods to determine oxidation of low-density lipoproteins. Methods Enzymol. 1994;233:425-441
34. Okazaki M, Ohno Y, Hara I. Rapid method for the quantitation of cholesterol in human serum lipoproteins by high performance liquid chromatography. Journal of biochemistry. 1981;89:879-887

## Claims

1. 3-hydroxyanthranilic acid (3-HAA) for use in the treatment of hyperlipidemia, in the prevention of a cardiovascular complication of hyperlipidemia or in the prevention of xanthomas.

2. 3-HAA for use according to claim 1, wherein said hyperlipidemia is selected from the group consisting of hypercholesterolemia, hypertriglyceridemia and combined (forms of) hyperlipidemia.

3. 3-HAA for use according to claim 1 or 2, wherein said hyperlipidemia is associated with low levels of high-density lipoprotein (HDL) in plasma.

4. 3-HAA for use according to anyone of the previous claims, wherein said cardiovascular complication of hyperlipidemia is atheroma formation.

5. 3-HAA for use according to anyone of claims 1 to 3, wherein said cardiovascular complication of hyperlipidemia is a clinical manifestation of atheroma formation, which clinical manifestation is chosen from myocardial infarction and/or heart failure.

6. 3-HAA for use according to any one of claims 4 to 5, for prevention of angina pectoris.

7. 3-HAA for use according to any one of claims 4 to 5, for prevention of ischemic stroke and/or transient ischemic attacks.

8. 3-HAA for use according to any one of claims 4 to 5, for prevention of peripheral ischemia, gangrene, renal impairment, aortic aneurysms, and/or critical limb ischemia caused by atherosclerosis.

## Patentansprüche

1. 3-Hydroxyantranilsäure (3-HAA) für die Verwendung bei der Behandlung von Hyperlipidämie, bei der Vorbeugung einer kardiovaskulären Komplikation von Hyperlipidämie oder bei der Vorbeugung von Xanthomen.

2. 3-HAA für die Verwendung gemäß Anspruch 1, wobei die Hyperlipidämie ausgewählt ist aus der Gruppe bestehend aus Hypercholesterolämie, Hypertriglceridämie und kombinierter (kombinierten Formen von) Hyperlipidämie.

3. 3-HAA für die Verwendung gemäß Anspruch 1 oder 2, wobei die Hyperlipidämie mit niedrigen Spiegeln von Lipoprotein mit hoher Dichte (HDL) im Plasma verbunden ist.

4. 3-HAA für die Verwendung gemäß einem der vorstehenden Ansprüche, wobei die kardiovaskuläre Komplikation von Hyperlipidämie Atheromentstehung ist.

5. 3-HAA für die Verwendung gemäß einem der Ansprüche 1 bis 3, wobei die kardiovaskuläre Komplikation von Hyperlipidämie eine klinische Manifestation von Atheromentstehung ist, welche klinische Manifestation ausgewählt ist aus Myokardinfarkt und/oder Herzinsuffizienz.

6. 3-HAA für die Verwendung gemäß einem der Ansprüche 4 bis 5 für die Vorbeugung von Angina pectoris.

7. 3-HAA für die Verwendung gemäß einem der Ansprüche 4 bis 5 für die Vorbeugung von ischämischem Schlaganfall und/oder transitorischen ischämischen Attacken.

8. 3-HAA für die Verwendung gemäß einem der Ansprüche 4 bis 5 für die Vorbeugung von peripherer Ischämie, Gangrän, Nierenschädigung, Aortenaneurysmen und/oder durch Atherosklerose verursachter kritischer Ischämie der Gliedmaßen.

## Revendications

1. Acide 3-hydroxyanthranilique (3-HAA) destiné à être utilisé dans le traitement d'une hyper-lipidémie, dans la prévention d'une complication cardiovasculaire d'une hyper-lipidémie ou dans la prévention de xanthomes.

2. 3-HAA destiné à être utilisé selon la revendication 1, dans lequel ladite hyper-lipidémie est choisie dans le groupe constitué par une hyper-cholestérolémie, une hypertriglycéridémie et des (formes de) hyper-lipidémies combinées.

3. 3-HAA destiné à être utilisé selon les revendications 1 ou 2, dans lequel ladite hyper-lipidémie est associée à des taux bas de lipoprotéines de haute densité (HDL) dans le plasma.

4. 3-HAA destiné à être utilisé selon l'une quelconque des revendications précédentes, dans lequel ladite complication cardiovasculaire d'une hyper-lipidémie est une formation d'athérome.

5. 3-HAA destiné à être utilisé selon l'une quelconque des revendications 1 à 3, dans lequel ladite complication cardiovasculaire d'une hyper-lipidémie est une manifestation clinique de formation d'athérome, laquelle manifestation clinique est choisie parmi un infarctus du myocarde et/ou une insuffisance cardiaque.

6. 3-HAA destiné à être utilisé, selon l'une quelconque des revendications 4 à 5, pour la prévention d'une angine de poitrine.

7. 3-HAA destiné à être utilisé, selon l'une quelconque des revendications 4 à 5, pour la prévention d'un accident vasculaire cérébral ischémique et/ou des ischémies cérébrales transitoires.

8. 3-HAA destiné à être utilisé, selon l'une quelconque des revendications 4 à 5, pour la prévention d'une ischémie périphérique, de la gangrène, d'une déficience rénale, d'anévrismes aortiques et/ou d'une ischémie critique des membres causée par l'athérosclérose.
